# EUROPEAN PATENT SPECIFICATION

(11) **EP 1 120 088 B1**
(45) Date of publication and mention of the grant of the patent: **29.04.2009**
(21) Application number: 00610015.0
(22) Date of filing: 28.01.2000
(51) Int. Cl.: A61B 17/12

(54) **An embolization device introducer**
Vorrichtung zur Einführung einer Vorrichtung zur intravaskulären Embolisierung
Dispositif d'introduction d'un dispositif d'embolisation

(43) Date of publication of application: 01.08.2001
(73) Proprietor: WILLIAM COOK EUROPE ApS, 4632 Bjaeverskov (DK); Cook Incorporated, Bloomington, IN 47402-0489 (US)
(72) Inventor: Klint, Henrik Sonderskov, 2800 Lyngby (DK)
(74) Representative: Indahl, Peter Jensen

(56) References cited:
- EP-A- 0 318 046
- EP-A- 0 737 487
- US-A- 5 373 619
- US-A- 5 419 339
- US-A- 5 645 064
- US-A- 5 725 534
- US-A- 5 759 161
- US-A- 5 800 455
- US-A- 5 984 877

## Description

The present invention relates to an embolization device introducer comprising an elongated delivery member (1) suitable for introduction through a catheter, said delivery member having a proximal section and a distal section (3), the distal section having a convection means for detachably mounting of an embolization device.

An embolization device introducer of this kind is known from US patent 5,725,534 describing that a threading coil is fixed on a rotationally symmetrical distal end portion of a guidewire core member. Controlled release of embolization coils is also known from WO 93/11823 which describes an embolization device introducer, which on a core at its distal end has two axially spaced soldered-on radial enlargements locking coil ends to the core. In US patent 5,122,136 a coil is connected with a guidewire through thin connecting areas which are electrolytically eroded away when the coil is positioned at the desired site. US patent 4,994,069 provides an adhesive fastening of the coil to the guidewire, and a release by heating of the adhesive layer with laser energy supplied through an optical fibre. WO 91/13592 describes an embolization device introducer with a coil bonded to the introducer by a metal-to-metal joint that can be broken electrolytically by applying a current to the introducer.

It is a problem common to all prior art embolization device introducers that the delivery member has a relatively high rigidity which renders difficult to advance the embolization coil to deployment sites without considerably affecting the vasculature. The delivery member presses heavily against the vascular walls in areas of tortuous paths. This problem is in particular pronounced in the case of embolization of aneurysms which have caused weakening of the wall strength of the vessel, and also pronounced in very narrow blood vessels in soft tissues, like in the brain.

The object of the present invention is to provide an embolization device introducer which can access vascular sites more easily for easy and secure placement of the embolization coil.

The embolization device introducer of the invention is accordingly characterized in that the delivery member comprises a helical winding of a multifilar row of wires extending from the connection means at the distal section to the proximal section of the delivery member in which the wires in the row are located in contact with each other, so as to keep the individual wire in a permanent helical shape without any further restraints than the remaining wires in the row.

By making the delivery member of at least a row of two or more wires, which row is helically wound with a pitch roughly corresponding to the aggregate width of the adjacent wires in the row, the wound wires transfer torque and also force components directed in the axial direction of the introducer to the distal end thereof, and this construction is found to give a very precise control of the embolization device advancement and positioning. When the delivery member is heavily bent the cross-section of the member maintains its circular shape. This is an distinct advantage over prior art tubular members which are deformed into oval shape when bent, and thus they are much more prone to kinking. The delivery member according to the present invention surprisingly maintains its capabilities for transferring torque and push when it follows a tortuous path involving two or more loops, and in curved areas the torque and push is mainly transmitted within the delivery member resulting in a favourably low influence on the vascular walls.

These capabilities are presumably a result of a kind of mutual interlocking of the individual wire stands in the row of wires. If one wire in the row has a tendency to kink or bend heavily under influence of the load applied to the delivery member, the other wires in the row keep said wire in place because they are all extending in a common helical course, which interlocks the wires. After advancement of the introducer to the desired deployment site, a rotational movement at the distal end of the delivery member is immediately transmitted into an almost identical rotational movement of the connection means (viz. about 1:1 torque transferral). Such an introducer is particularly useful in association with the connection means being designed for detachment by unscrewing from the embolization device, because the rotation of the delivery member during unscrewing will cause only negligent influence on the vasculature, and the embolization device can thus easily be kept exactly at the desired position during the detachment, and furthermore there is obtained a very precise control of the detachment when e.g. three turns at the proximal end imediately results in an identical three turn rotation at the distal end of the delivery member.

Due to the very high flexibility, pushability and torqueability and the ability of the construction to maintain each of these three characteristics even when set in a very tortuous pattern involving two or more tight loops, the embolization device introducer can be of use in very small and distant vessels such as deep brain sites accessed by intercranial catheterization.

In one embodiment the row of wires is made up of from 2 to 12 helically wound wires, preferably of from 4 to 8 helically wound wires. By using several wires their aggregate width can be adapted to correspond to the desired pitch for the given diameter of the embolization device introducer. A row of more than 12 wires has a tendency to buckle when the wires are helically wound in the common winding operation. For wires of round cross-sectional shape a number of from 4 to 8 wires in the row is preferred, but for flat wires or wires of oval shape two or three wires in a row can be more suitable.

In order to promote uniform and well-defined characteristics of the embolization device introducer along its length, the wires in the row can be located closely next to each other so that they mutually contact each other almost continuously. In this manner a possible deflection of a single wire strand is reduced to a minimum by the other wires in the row. As the wires in the row are wound into a helical course in a common movement there can be a interstice between the turns of the row of wires.

In an embodiment the wires in said row have a pitch angle in the range of 26°-76°, preferably a pitch angle in the range of 40°-65°. Although it is possible to use other pitch angles, angles chosen in these ranges provide a balanced solution to the requirements for the desired high flexibility, high column strength and fine torqueability. The inner range of 40°-65° is in particular useful for placement of a embolization device in very distant, small sized vessels, such as in blood vessels in the brain, whereas the subrange of 35°-40° can be applicable when very high flexibility is a requirement, and the subrange of 70°-76° can be applicable when very high pushability is a requirement. It is of course possible to choose different pitch angles in different segments of the delivery member.

At the time of performing the winding operation of the delivery member, the individual wires in the row wound in the helical pattern have preferably a mainly circular cross-section. This facilitates the winding operation because twisting of a wire does not result in disorder in the row.

It is possible to promote the flexibility of the embolization device introducer by machining the wires in said row to a lesser outer diameter, e.g. by grinding, at a region of the delivery member. The region can extend along the whole length of the delivery member, so that it is given a very precise outer dimension by the machining. In another embodiment the region is a distal region machined to a tapering shape with decreasing outer diameter in the distal direction causing the embolization device introducer to have an increasing flexibility towards the distal end which promotes introduction into very diminutive vessels. The reduced cross-sectional area of the wires produced by the machining greatly increases the bending flexibility of the delivery member without sacrificing its ability to transfer torque.

It is preferred that the embolization device introducer at least in a 30 cm long distal area has a maximum outer diameter of at the most 1.0 mm. A maximum diameter of 1 mm in the part of the embolization device introducer advanced through the vascular system allows for a straightforward percutaneous introduction by the Seldinger technique and easy navigation through the curves in the larger vessels. Coils having the relatively large diameters in the range of 0.7 to 1.0 mm are suitable for embolization in larger vessels, and in particular at locations where the blood flow rate is high, e.g. due to a malformation or trauma. A maximum diameter of maximum 1.00 mm allows introduction into quite fine and diminutive vessels such as into the external and internal carotid arteries. It is further possible to keep the maximum outer diameter at at the most 0.75 mm, and preferably at the most 0.50 mm, which makes it possible to easily advance the embolization device introducer into e.g. the liver or other soft tissue areas, and by keeping the maximum outer diameter below 0.30 mm in a distal end area having a length of at least 10 cm even the most distant vascular regions are accessible for occlusion by the embolization device introducer.

In a further embodiment the number of wires in said helically wound row of wires varies along the length of the delivery member. This can be attained by reducing, during the winding operation, the number of wires in the row. The lower number of wires in the row can be utilized for winding the wires with a larger pitch angle which increases the flexibility of the delivery member. In this case it is preferred that the number of wires diminishes in the distal direction so that the softness of the introducer increases without any change of material and without bonding together several separate delivery member segments.

The introducer according to the present invention is applicable with all kinds of connection means, but in order to use well-proven means the connection means is preferably selected among the group of an electrolytically erodable means, a heat erodable means, a latch, a coupling, a threading coil, a thread, a deflatable balloon, and a hydraulically or pneumatically activated gripper means.

In a preferred embodiment the delivery member has in its distal end portion a central core with a blade-shaped portion, and the connection means is a threading coil which is fixed to the central core at least at the edges of the blade-shaped portion. The blade-shaped portion carrying the threading coil is much more flexible and easy to bend in the thickness direction of the blade than in the direction of the width where the blade dimension is the largest. The blade-shaped portion is a distal end portion of the central core and if it is subjected to a torque the central core twists. When the delivery wire is advanced and has to pass through a curvature, the blade-shaped portion touches the inner wall of the lumen and is subjected to a torque until the blade-shaped portion has turned itself with the direction of width transverse to the curvature. The result is that the bending occurs in the thickness direction which is most flexible. The fixation of the threading coil at the edges provides control of the positioning of the threads so that the un-threading of the embolization device is very smooth-running.

The connection means can be made of radiopaque material in order to be discerned on an image screen by the radiologist or neuroradiologist that introduces the detachable embolization device into the vascular system of a patient, but in order to be seen clearly the radiopaque area ought to have relatively large dimensions. This can be obtained by positioning a radiopaque marker at a predefined first distance, such as about 3 to 3.5 cm, proximal to the distal termination of the connection means. In this embodiment the connection means in itself need not be radiopaque, because the marker is clearly seen and the radiologist is aware that the embolization device is positioned said first distance ahead of the marker.

The invention will now be explained in further detail below with reference to the very schematic drawings of examples of preferred embodiments, in which
Fig. 1 depicts a partial view of a delivery member of an embolization device introducer according to the present invention,
Fig. 2 is a sketch of the introducer ready for disengaging an embolization device,
Fig. 3 is an enlarged illustration of the distal end of the delivery member with an embolization device during placed i a catheter,
Figs. 4, 5 and 11 are partial views of the delivery member of another embodiments,
Figs. 6-8 are views of different embodiments providing increased flexibility in the distal end area of the delivery member,
Fig. 9 is a view of the winding of a row of wires into the delivery member, and
Fig. 10 illustrates delivering an embolization device by the embolization device introducer.

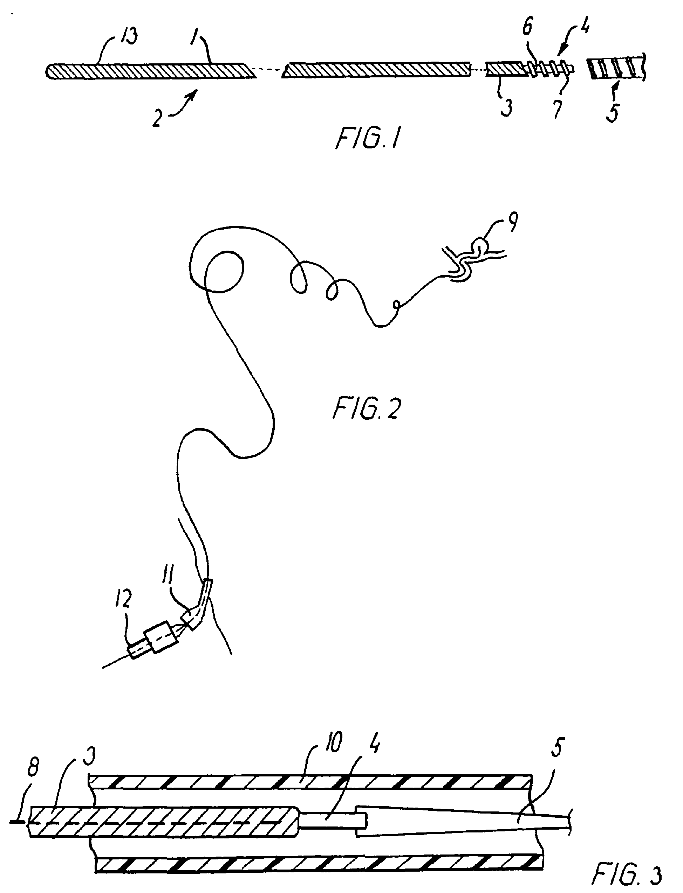

A delivery member 1 of an embolization device introducer 2, Fig. 1, has a length in the range of 50 to 250 cm and a diameter in the range of 0.08 to 2 mm, depending on the relevant field of application. In a distal section 3 the delivery member has a connection means 4 for an embolization device 5.

In the following description of several embodiments the same numerals are used to denote features of the same kind.

In one embodiment the connection means 4 comprises a central core 6 of stainless steel, nitinol, or another suitable material and a threading coil 7. The central core 6 has at its distal end a bladeshaped portion with a blade thickness and a blade width, which is more than twice as large as the blade thickness. The threading coil 7 is fixed onto the blade-shaped portion, e.g. by soldering, welding, brazing or gluing. The threading coil wire can be of stainless steel and can have a wire diameter in the range of 0.02 to 0.12 mm, typically a diameter of about 0.06 to 0.075 mm. The wire is set with a pitch corresponding to or being larger than twice the thickness of the wire so that a mating threading in the proximal end of the detachable embolization device 5 can be threaded into and out of threading coil 7. The outer diameter of the threading coil can, for superselective use, be in the range of 0.08 to 1 mm, and typically from 0.20 mm to 0.45 mm.

Other embodiments of the connection means 4 include a connecting area, which is eroded away by applying current or heat when the embolization device is positioned at the desired site, or a latch or a coupling providing a geometrical locking, such as a bayonet coupling, two mating parts held together by a thread that can be pulled out for detachment of the embolization device, or a deflatable balloon positioned inside a tubular proximal end area of the embolization device. Other embodiments of threads can also be used, such as spaced ball-like enlargements on the central member, a helix-shaped groove cut into a cylindrical or conical distal end part on delivery member 1. These kinds of connection means are well known in the art (e.g. from EP-A-720 838; US patent 5,217,484; WO 94/06503; WO 94/06502; WO94/00104; and EP-A 717 969), and in Fig. 3 such a connection means 4 is shown in a general manner, and an activation member 8 is shown to extend inside the delivery member. The activation member can be e.g. the above mentioned thread to be pulled out, an optical fibre, an electric wire etc.

The embolization device can be a Gianturco stainless steel coil of traditional design, or coils with a regular helical shape or irregular coil shape as described in US patent 4,994,069, US patent 5,122,136, WO 93/06883, WO 94/11051, WO 94/07560, WO 94/10936, WO95/25480, DE-295 18 932-U1, WO 96/18343, EP 0 623 012, or the embolization device can be of a random matrix shape as described in US patent 4,994,069 and WO 94/09705. The embolization device can also be of a regular linear shape as described in WO 98/09570. The embolization device can also be called an occlusion device.

Referring now to Figs. 2 and 3 placement of the embolization device in an aneurism 9 will be described. A catheter is introduced percutaneously through a fitting 11 by the Seldinger technique and advanced transluminally in a well-known manner along a suitable path until the distal end of the catheter is located in the neck of the aneurism. Then an introducer 2 with the embolization device mounted on the delivery member 1 is inserted into the catheter and pushed forwards until the embolization device is pushed out of the catheter and is in the desired deployment position in aneurysm 9. So positioned, the delivery member extends along a complexly curved path. Then the embolization device is released from connection means 4. This can e.g. be done by activating member 8 or by rotating the proximal end of the delivery member with the aid of a pin vice 12 which is fixed onto a proximal section 13 of delivery member 1.

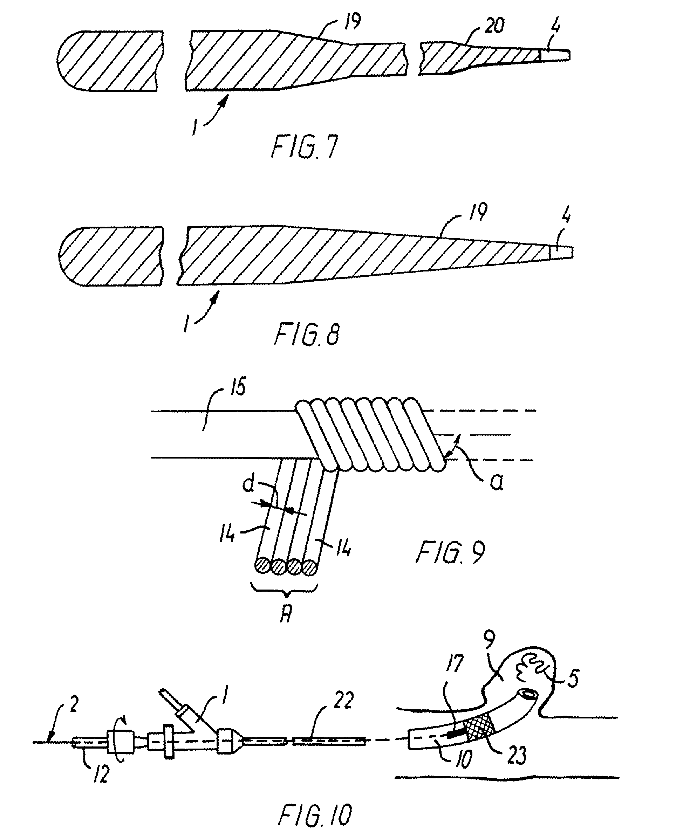

Delivery member 1 is made of a first helically wound multifilar row A of wires 14 (Fig. 9). The wires 14 used in the helically wound multifilar row are of a linear elastic material, such as stainless steel, titanium or tantalum, or they are made of a superelastic alloy, such as Nitinol. Preferably, the wires have an ultimate tensile strength in the range of 1800-2700 N/mm² but lower or higher values are also possible. The delivery member is made by placing a group of from two to twelve wires in a row A next to each other, where the number of wires is chosen e.g. according to the desired pitch angle and wire diameter, whereafter the group of wires is wound. Because a row of wires is wound, the individual wire is restricted in movement by the other wires and is plastically deformed into a permanent helical shape which is kept without any further restraints than the remaining wires in the row. The winding can be done on the inside end of a tubular support member where the row of wires are inserted at said end by rotating and simultaneously pushing the wires against the inside of the support. The wound wire then exits at the other end of the support. This produces a delivery member with a very precise outer diameter.

Alternatively, the winding operation can take place about a mandrel 15. Fig. 9 depicts winding of a row A of four identical wires 14. After the winding the mandrel with the coiled wires can be subjected to heat treatment in order to remove residual stresses from the wires. As an example the heat treatment can last for about two hours in an oven at a temperature of about 500°C. Generally, the temperature can be in the range of 400-600°C and the holding time at the temperature can last for many hours, such as up till 20 hours or more. When a mandril is used it can ceonveniently be removed after the heat treatment. The resulting helically wound multifilar row of wires maintain their mutual position even when heavily torqued, bent or pushed, presumably because each single wire is supported by the contiguous wires in the row.

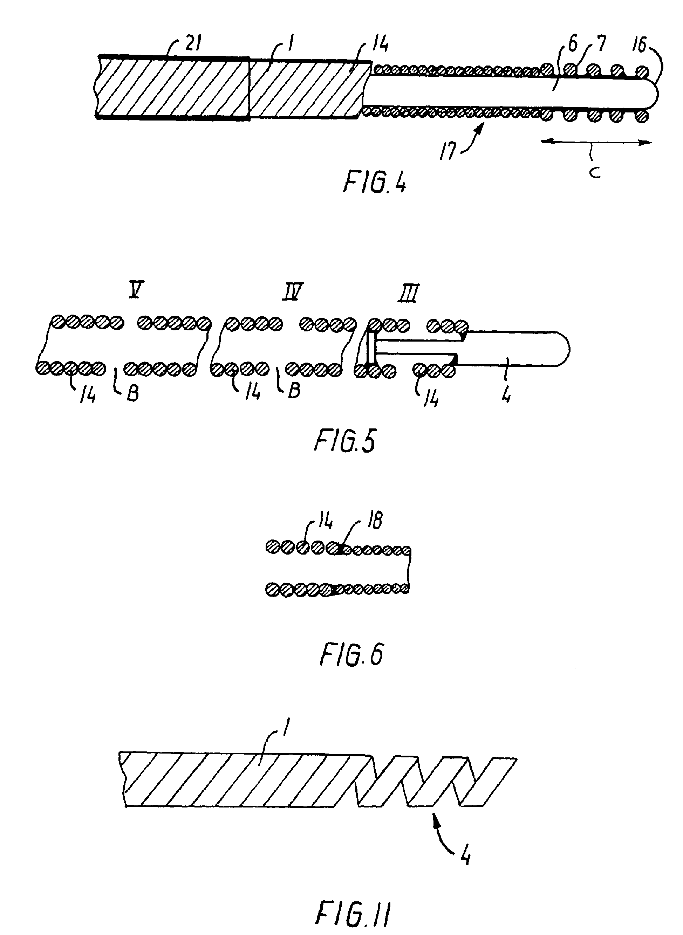

The winding operation can be effected so that the windings are touching each other, but preferably it is performed so that a sligt interstice B is present between the turns (Fig. 5). The interstice facilitates bending of the body portion portion in thight turns.

The size of the pitch angle a depends on the diameter of the wires, the diameter of the delivery member 1 and the number of wires in the row. The most preferred pitch angle a for the delivery member is in the range of 40° - 65°. However, the combination of torque-transferral, pushability and transverse flexibility is normally well-balanced for pitch angles in the range of 50-68°. The diameter d of the wire is typically in the range of 0.03-0.75 mm, and preferably in the range of 0.15-0.45 mm.

In order to make the tip portion of the delivery member more visible on a screen, it is desirable to use some kind of a radiopaque marker 17 of radiopaque material, such as platinum or gold. It can be of annular shape and be located at a predetermined distance c from the distal end 16, Fig. 4. The marker can be of platinum wire inserted into delivery member 1 in distal extension of wires 14, or it can be a separate member such as a platinum or gold ring. A catheter 10 used when advancing the introducer can also have a radiopaque marker 23 located at such a distance from the distal end of the catheter that the embolization device is in position for release when the marker 17 has been advanced to be positioned at marker 23 (Fig. 10).

In the embodiment illustrated in Fig. 5 the number of wires 14 in said helically wound row of wires varies along the length of the delivery member. During the winding operation the number of wires in the row is reduced one by one at the points in time where individual segments having a constant number of wires have obtained their desired lengths. The segments marked V, IV and III have five, four and three wires, respectively, in the row. Each time a wire is left out of the row, the pitch gets shorter and the pitch angle grows resulting in a even more flexible consecutive segment. The advantage of this embodiment is that the wires extending into the distal end segment are continuous from the distal end to the proximal end of the delivery member, thus avoiding any need for joining the various segments. It is possible to secure the thread ends of the discontinous wires onto the other wires, such as by welding, soldering, etc.

The delivery member can be made with uniform diameter throughout its length. In case the delivery member is to have diminishing diameter towards the distal end, a prefabricated delivery member of uniform diameter can be grinded to the desired dimensions.

As an alternative or supplement to grinding the delivery member can be composed of several segments in which the wires have mutually different diameters and cross-sectional areas. In a proximal section 13 the wires can have a larger diameter than the wires in a distal section 3. The sections or segments can be joined together in axial extension by laser welds 18 as depicted in Fig. 6, by soldering, by bracing or in another manner such as mutual geometrically locking of the wires in the segments or by mechanical locking, such as press-fitting one segment into the lumen of the other segment, or binding the segments in axial extension with threads or suture.

A grinding procedure can also be used to produce one or more tapered segments 19, 20 in delivery member 1. The taper can extend along a substantial length of the delivery member to produce a gradually increasing flexibility. In the tapered segment the outer diameter of the delivery member 1 diminishes towards the distal end. Due to the taper the delivery member obtains a gradually increasing transverse flexibility and a higher softness, but column stength and torque are nevertheless surprisingly transferred to the distal end.

In the follwing some examples of delivery members made according to the invention are described.

### EXAMPLE 1:

A delivery member was made of a helically wound row of 4 wires of 0.30 mm wire diameter d. The delivery member had initially an outside diameter of 0.90 mm. The delivery member was set in a complex curved shape involving three consecutive loops of a loop diameter of 24 mm axially separated by two loops of a loop diameter of 18 mm and a number of further turns representative of a complex vascular structure. Then the proximal section of the delivery member was manipulated and it proved to be easily pushed forward and retracted as well as easily torqued.

### EXAMPLE 2:

A delivery member was made of a first helically wound row of 8 wires of 0.075 mm wire diameter d. The winding was made with an outside diameter of 0.25 mm. The delivery member had a length of 160 cm. When tested the delivery member showed no problems. After placing the delivery member in a very complex pattern involving several sharp turns, the distal end could be rotated in a 1:1 relationship with a rotation of the proximal end of the delivery member.

When large lumen vessels are to be traversed in order to enter the vasculature near the target site, it can be an advantage to stiffen the delivery member by inserting it through a cannula 22 (Fig. 10) or another kind of a more rigid structure.

The helically wound row of wires in the delivery member makes it possible to manufacture the connection means as an integral part of the delivery member. This can be done by removing one or several of the wires in wires are very diminutive they can be cut, e.g. by a laser beam or manually with a tool under microscope. If required a thread cutter tool or a thread shaping tool can be used to set the remaining wire(s) with the desired pitch corresponding to the pitch on the mating coupling member on the embolization device. The resulting unitary delivery member has in its distal end only the wires which extend towards the proximal end.

Individual features of the various embodiments can be combined into further embodiments according to the present invention.

## Claims

1. An embolization device introducer comprising an elongated delivery member (1) suitable for introduction through a catheter, said delivery member having a proximal section and a distal section (3), the distal section having a connection means for detachably mounting of an embolization device, **characterized in that** the delivery member (1) comprises a helical winding of a multifilar row (A) of wires (14) extending from the connection means at the distal section to the proximal section of the delivery member in which the wires (14) in the row (A) are located in contact with each other, so as to keep the individual wire in a permanent helical shape without any further restraints than the remaining wires in the row.

2. An embolization device introducer according to claim 1, **characterized in that** the wires are made from a wire material from the group comprising stainless steel,, titanium, tantalum and a superelastic alloy.

3. An embolization device introducer according to claim 1, **characterized in that** the wires are made from a wire material having an ultimate tensile strength in the range from 1800 to 2700 N/mm².

4. An embolization device introducer according to any one of the claims 1-3, **characterized in that** the row (A) of wires is made up of from 2 to 12 helically wound wires (14), preferably of from 4 to 8 helically wound wires (14).

5. An embolization device introducer according to any one of claims 1-4, **characterized in that** the wires (14) have a pitch angle in the range of 26°-76°, preferably a pitch angle in the range of 40°-65°.

6. An embolization device introducer according to any one of claims 1-5, **characterized in that** the delivery member (1) is wound of wires (14) having a mainly circular cross-section.

7. An embolization device introducer according to any one of the claims 1-6, **characterized in that** the wires (14) in said row (A) have been machined to a lesser outer diameter, e.g. by grinding, in a region of the delivery member (1).

8. An embolization device introducer according to claim 7, **characterized in that** said region is a distal region (19, 20) having a tapering shape with decreasing outer diameter in the distal direction.

9. An embolization device introducer according to any one of the claims 1-8, **characterized in that** the delivery member (1) at least in a 30 cm long distal segment has a maximum outer diameter of at the most 1.0 mm.

10. An embolization device introducer according to any one of the claims 1-9, **characterized in that** the delivery member (1) has a 30 cm long distal segment having a maximum outer diameter of less than 0.75 mm, preferably at the most 0.50 mm.

11. An embolization device introducer according to any one of the claims 1-10, **characterized in that** the delivery member (1) has a distal segment of a length of at least 15 cm which has a maximum outer diameter of less that 0.30 mm.

12. An embolization device introducer according to any one of the claims 1-11, **characterized in that** the connection means (4) is selected among the group of an electrolytically erodable means, a heat erodable means, a latch, a coupling, a threading coil, a thread, a deflatable balloon, and a hydraulically or pneumatically activated gripper means.

13. An embolization device introducer according to any one of the claims 1-12, **characterized in that** the delivery member (1) in its distal end portion has a central core with a blade-shaped portion, and that said connection means (4) is a threading coil which is fixed to the central core at least at the edges of the blade-shaped portion.

14. An embolization device introducer according to any one of claims 1-13, **characterized in that** a radiopaque marker (23) is positioned at a predefined first distance, such as about 3 to 3.5 cm, proximal to the distal termination of said connection means (4).

## Patentansprüche

1. Vorrichtung zur Einführung einer Vorrichtung zur Embolisierung mit einem langen Lieferglied (1) zur Einführung durch einen Katheter, das ein vorderes Teilstück und ein distales Teilstück (3) aufweist, wobei das distale Teilstück Mittel zur abtrennbaren Befestigung einer Vorrichtung zur Embolisierung aufweist, **dadurch gekennzeichnet, dass** das Lieferglied (1) eine schraubenlinienförmige Windung einer multifilaren Reihe (A) von Drähten (14) umfasst, die sich von den Mitteln zur Befestigung am distalen Teilstück zum vorderen Teilstück des Liefergliedes erstreckt, indem die sich berührenden Drähte (14) der Reihe (A) derart angeordnet sind, dass der einzelne Draht ohne irgendwelche weiteren Einschränkungen als die übrigen Drähte in der Reihe ständig in einer schraubenlinienförmigen Form gehalten sind.

2. Vorrichtung zur Einführung einer Vorrichtung zur Embolisierung nach Anspruch 1, **dadurch gekennzeichnet, dass** die Drähte aus einem Drahtmaterial der Gruppe, die aus Edelstahl, Titan, Tantal und einer superelastischen Metalllegierung besteht, hergestellt sind.

3. Vorrichtung zur Einführung einer Vorrichtung zur Embolisierung nach Anspruch 1, **dadurch gekennzeichnet, dass** die Drähte aus einem Drahtmaterial mit einer äußersten Zugfestigkeit im Bereich von 1800 bis 2700 N/mm² hergestellt sind.

4. Vorrichtung zur Einführung einer Vorrichtung zur Embolisierung nach einem der Ansprüche 1 bis 3, **dadurch gekennzeichnet, dass** die Reihe (A) von Drähten aus 2 bis 12 schraubenlinienförmig gewundenen Drähten (14), bevorzugt aus 4 bis 8 spiralförmig gewundenen Drähten (14), hergestellt ist.

5. Vorrichtung zur Einführung einer Vorrichtung zur Embolisierung nach einem der Ansprüche 1 bis 4, **dadurch gekennzeichnet, dass** die Drähte (14) einen Steigungswinkel im Bereich von 26° bis 76°, bevorzugt einen Steigungswinkel im Bereich von 40° bis 65°, aufweisen.

6. Vorrichtung zur Einführung einer Vorrichtung zur Embolisierung nach einem der Ansprüche 1 bis 5, **dadurch gekennzeichnet, dass** das Lieferglied (1) aus Drähten (14) mit einem hauptsächlich kreisförmigen Durchmesser gewunden ist.

7. Vorrichtung zur Einführung einer Vorrichtung zur Embolisierung nach einem der Ansprüche 1 bis 6, **dadurch gekennzeichnet, dass** die Drähte (14) in der Reihe (A), zum Beispiel durch Schleifen, in einem Bereich des Liefergliedes (1) maschinell auf einen kleineren äußeren Durchmesser gebracht sind.

8. Vorrichtung zur Einführung einer Vorrichtung zur Embolisierung nach einem der Ansprüche 1 bis 7, **dadurch gekennzeichnet, dass** der besagte Bereich ein distaler Bereich (19, 20) ist, der eine konische Form mit in der distalen Richtung abnehmendem Außendurchmesser aufweist.

9. Vorrichtung zur Einführung einer Vorrichtung zur Embolisierung nach einem der Ansprüche 1 bis 8, **dadurch gekennzeichnet, dass** das Lieferglied (1) zumindest in einem 30 cm langen distalen Segment einen maximalen Außendurchmesser von höchstens 1,0 mm aufweist.

10. Vorrichtung zur Einführung einer Vorrichtung zur Embolisierung nach einem der Ansprüche 1 bis 9, **dadurch gekennzeichnet, dass** das Lieferglied (1) ein 30 cm langes distales Segment mit einem maximalen Außendurchmesser von weniger als 0,75 mm, bevorzugt höchstens 0,50 mm, aufweist.

11. Vorrichtung zur Einführung einer Vorrichtung zur Embolisierung nach einem der Ansprüche 1 bis 10, **dadurch gekennzeichnet, dass** das Lieferglied (1) ein zumindest 15 cm langes distales Segment mit einem maximalen Außendurchmesser von weniger als 0,30 mm aufweist.

12. Vorrichtung zur Einführung einer Vorrichtung zur Embolisierung nach einem der Ansprüche 1 bis 11, **dadurch gekennzeichnet, dass** die Mittel zum Befestigen (4) aus der Gruppe bestehend aus elektrolytisch erodierbaren Mitteln, durch Hitze erodierbaren Mitteln, Verschlüssen, Kupplungen, Gewindewicklungen, Gewinden, entleerbare Ballonen und hydraulisch oder pneumatisch aktivierten Mitteln zum Greifen ausgewählt sind.

13. Vorrichtung zur Einführung einer Vorrichtung zur Embolisierung nach einem der Ansprüche 1 bis 12, **dadurch gekennzeichnet, dass** das Lieferglied (1) am distalen Endteilstück einen zentralen Kern mit einem klingenförmigen Teilbereich aufweist, und dass die Mittel zum Befestigen (4) aus einer Gewindewicklung bestehen, die zumindest an den Kanten des klingenförmigen Teilbereichs mit dem zentralen Kern verbunden sind.

14. Vorrichtung zur Einführung einer Vorrichtung zur Embolisierung nach einem der Ansprüche 1 bis 13, **dadurch gekennzeichnet, dass** eine röntgenfähige Markierung (23) in einer vorbestimmten Entfernung wie etwa 3 bis 3,5 cm vor dem distalen Abschluss der Mittel zum Befestigen (4) verankert ist.

## Revendications

1. Dispositif d'introduction d'un dispositif d'embolisation comprenant un élément de pose allongé (1) approprié pour l'introduction par l'intermédiaire d'un cathéter, ledit élément de pose ayant une section proximale et une section distale (3), la section distale ayant des moyens de raccordement pour monter de manière amovible un dispositif d'embolisation, **caractérisé en ce que** l'élément de pose (1) comprend un enroulement hélicoïdal d'une rangée multifilaire (A) de fils (14) s'étendant à partir des moyens de raccordement au niveau de la section distale jusqu'à la section proximale de l'élément de pose, dans lequel les fils (14) dans la rangée (A) sont positionnés en contact les uns avec les autres, afin de maintenir le fil individuel selon une forme hélicoïdale permanente sans aucune autre contrainte que les fils restants dans la rangée.

2. Dispositif d'introduction d'un dispositif d'embolisation selon la revendication 1, **caractérisé en ce que** les fils sont composés à partir d'un matériau de fil du groupe comprenant l'acier inoxydable, le titane, le tantale et un alliage super élastique.

3. Dispositif d'introduction d'un dispositif d'embolisation selon la revendication 1, **caractérisé en ce que** les fils sont composés à partir d'un matériau de fil ayant une résistance à la traction ultime de l'ordre de 1800 à 2700 N/mm².

4. Dispositif d'introduction d'un dispositif d'embolisation selon l'une quelconque des revendications 1 à 3, **caractérisé en ce que** la rangée (A) de fils est composée de 2 à 12 fils (14) enroulés de manière hélicoïdale, de préférence de 4 à 8 fils (14) enroulés de manière hélicoïdale.

5. Dispositif d'introduction d'un dispositif d'embolisation selon l'une quelconque des revendications 1 à 4, **caractérisé en ce que** les fils (14) ont un pas d'hélice de l'ordre de 26°-76°, de préférence un pas d'hélice de l'ordre de 40°-65°.

6. Dispositif d'introduction d'un dispositif d'embolisation selon l'une quelconque des revendications 1 à 5, **caractérisé en ce que** l'élément de pose (1) est enveloppé de fils (14) ayant une section transversale principalement circulaire.

7. Dispositif d'introduction d'un dispositif d'embolisation selon l'une quelconque des revendications 1 à 6, **caractérisé en ce que** les fils (14) dans ladite rangée (A) ont été usinés à un diamètre externe plus petit, par exemple par meulage, dans une région de l'élément de pose (1).

8. Dispositif d'introduction d'un dispositif d'embolisation selon la revendication 7, **caractérisé en ce que** ladite région est une région distale (19, 20) ayant une forme progressivement rétrécie avec le diamètre externe qui diminue dans la direction distale.

9. Dispositif d'introduction d'un dispositif d'embolisation selon l'une quelconque des revendications 1 à 8, **caractérisé en ce que** l'élément de pose (1) dans un segment distal d'au moins 30 cm de long, a un diamètre externe maximum de 1,0 mm au maximum.

10. Dispositif d'introduction d'un dispositif d'embolisation selon l'une quelconque des revendications 1 à 9, **caractérisé en ce que** l'élément de pose (1) a un segment distal de 30 cm de long ayant un diamètre externe maximum inférieur à 0,75 mm, de préférence de 0,50 mm au maximum.

11. Dispositif d'introduction d'un dispositif d'embolisation selon l'une quelconque des revendications 1 à 10, **caractérisé en ce que** l'élément de pose (1) a un segment distal d'une longueur d'au moins 15 cm qui a un diamètre externe maximum inférieur 0,30 mm.

12. Dispositif d'introduction d'un dispositif d'embolisation selon l'une quelconque des revendications 1 à 11, **caractérisé en ce que** les moyens de raccordement (4) sont choisis dans le groupe comprenant des moyens pouvant être érodés de manière électrolytique, des moyens pouvant être érodés à la chaleur, un verrou, un couplage, une bobine de filetage, un filetage, un ballonnet dégonflable, et des moyens de préhension activés de manière hydraulique ou pneumatique.

13. Dispositif d'introduction d'un dispositif d'embolisation selon l'une quelconque des revendications 1 à 12, **caractérisé en ce que** l'élément de pose (1) dans sa partie d'extrémité distale a un noyau central avec une partie en forme de lame, et **en ce que** lesdits moyens de raccordement (4) sont une bobine de filetage qui est fixée sur le noyau central au moins au niveau des bords de la partie en forme de lame.

14. Dispositif d'introduction d'un dispositif d'embolisation selon l'une quelconque des revendications 1 à 13, **caractérisé en ce qu'**un marqueur radio-opaque (23) est positionné à une première distance prédéfinie, telle qu'environ 3 à 3,5 cm, proximale par rapport à la terminaison distale desdits moyens de raccordement (4).
